# FASCICULE DE BREVET EUROPEEN

(11) **EP 4 196 040 B1**
(45) Date de publication et mention de la délivrance du brevet: **05.06.2024**
(21) Numéro de dépôt: 21756019.2
(22) Date de dépôt: 26.07.2021
(51) Int. Cl.: A61B 46/10

(54) **DISPOSITIF DE HOUSSAGE STERILE ET DE JOINT TOURNANT**
STERILE ABDECKUNGSVORRICHTUNG MIT DREHGELENK
STERILE COVERING DEVICE WITH A ROTATING JOINT

(30) Priorité: 17.08.2020 FR 2008526
(43) Date de publication de la demande: 21.06.2023
(73) Titulaire: STERLAB, 06220 Vallauris (FR)
(72) Inventeur: MARTEL, Paul, 83700 St-Raphaël (FR)
(74) Mandataire: Novagraaf Technologies
(86) Numéro de dépôt international: PCT/FR2021/051395
(87) Numéro de publication internationale: WO 2022/038321

(56) Documents cités:
- US-A1- 2010 292 707

## Description

### Domaine technique de l'invention

La présente invention concerne un nouveau dispositif de houssage de protection stérile et de joint tournant utilisé dans le domaine médical et plus particulièrement dans les salles d'opérations des hôpitaux et cliniques.

En effet en milieu hospitalier, un certain nombre de normes en matière d'hygiène doivent être respectées afin de ne pas transmettre de maladies au bloc opératoire. Or certains appareils utilisés en milieu hospitalier sont très compliqués et difficiles à stériliser car ils sont d'une part très fragiles (avec souvent beaucoup d'électronique) et très chers et d'autre part souvent très gros et nécessitant une désinfection particulière. De plus sur le long terme, les produits désinfectants pourraient largement endommager ces appareils.

C'est pourquoi les housses de protection stériles à usage unique, qui sont beaucoup moins onéreuse qu'une désinfection classique utilisée pour du matériel chirurgical, sont la solution idéale pour une stérilité optimale tout en conservant la qualité des appareils utilisés et en préservant leur usage sur le long terme. Elles permettent un gain considérable de temps et de dépenses, surtout lorsque l'on connaît le manque de personnel en milieu hospitalier.

Par ailleurs la robotique médicale, en particulier lors d'intervention chirurgicale, se développe de plus en plus et met en oeuvre des organes, souvent des bras, pouvant effectuer des déplacements ou des rotations selon les trois axes.

Une des applications de l'invention est ainsi l'usage, mais l'invention n'est bien sûr pas limité à celui-ci, d'un robot télémanipulateur pour introduire, dans le corps d'un patient, une gaine ou une fibre, en général semi-rigide, dans un conduit naturel ou artificiel, tel qu'une veine ou une artère pour des interventions cardiaques, ou l'urètre, ou une gaine d'accès préalablement mise en place pour servir de guide pour y introduire ensuite par exemple un outil d'intervention, et laquelle fibre ou gaine devant pouvoir tourner plusieurs fois sur elle-même .

### Arrière-plan technique

Il existe ainsi pour protéger de tels robots et appareils destinés aux blocs opératoires une large variété de housses de protection et drapage, tels que celles proposées par des sociétés comme en France CG Medical, EDM Imaging, IMMED... (dont on peut consulter librement les catalogues disponibles sur Internet), qui sont adaptées à tous types et à toutes marques d'appareils.

Ces housses de protection stériles permettent ainsi de lutter contre la transmission d'agents pathogènes. Comme indiqué ci-dessus, elles font parties des solutions rapides et efficaces pour assurer la stérilité des salles d'opérations même avec l'usage d'appareils et de robots qui eux sont compliqués et difficiles à stériliser : elles permettent donc de protéger ces robots et appareils et par voie de conséquence les patients. Mais si la mise en place de ce houssage ne pose pas de difficultés majeures pour les appareils sans mouvement propre ou ne comportant que des parties se déplaçant axialement, il n'en est pas de même pour les parties liées entre elles par un joint tournant qui leur permet de tourner les unes par rapport aux autres : certes en général, ce problème n'est pas insurmontable quand les rotations s'effectuent à+/- 180 ° au maximum par rapport à leur position initiale de référence, car il suffit alors de laisser une surface excédentaire suffisante à la housse au niveau du joint tournant soit par un pliage en accordéon soit par une augmentation de la lumière de la housse.

Cependant le problème du houssage devient critique et impossible à résoudre avec les housses et les joints actuels lorsque la rotation s'effectue sur un tour complet ou plus car la déformation de la housse ne permet plus de suivre la partie tournante par rapport à l'autre.

Ce houssage peut être réalisé également avec une ou plusieurs housses collées ou assemblées les unes aux autres pour un même appareil : ces housses en au moins deux parties sont solidaires entre elles par tout moyen (colle, ruban autocollant...) et dont par exemple l'une peut s'adapter sur un support de faible diamètre tandis que l'autre peut enserrer une pièce de plus grand diamètre placée à l'extrémité du support (les housses pour bras d'amplificateur de brillance en sont un exemple). Mais ces dispositifs de houssage en plusieurs parties ne permettent pas non plus comme indiqués précédemment d'effectuer plus d'une rotation complète d'une partie par rapport à l'autre et aucune solution n'est proposée à ce jour pour cela.

Dans les publications de brevets et demandes de brevet, on ne relève du reste que des dispositifs de protection de joint entre des parties mobiles les unes par rapport aux autres qui soit ne sont pas à usage unique (et donc nécessitent de les stériliser eux-mêmes ce que l'on veut justement éviter) soit ne protègent pas l'ensemble des parties mobiles et du joint tournant qui les relient (et qui nécessitent donc qu'au moins la partie non protégée soit stérilisée, ce que l'on veut également éviter), et qui sont en effet :
- soit mécaniques et solidairement liées d'une façon permanente à l'appareil comme dans le brevet EP3338668 déposé par la sté Ethicon le 21 décembre 2017 sur un « agencement de couverture de protection pour une interface de joint entre une mâchoire mobile et un arbre d'actionneur d'un instrument chirurgical »
- soit souples et séparant une partie stérile non protégée et une partie non stérile protégée par une housse, comme dans la demande de brevet EP437004 déposée par la sté allemande Effner Gmbh le 15 novembre 1990 qui enseigne des dispositifs pour protéger un instrument chirurgical, en particulier un endoscope, contre la contamination, comprenant une housse tubulaire qui est délimitée par une ouverture d'insertion et une ouverture de sortie comportant une membrane élastique, dont le bord extérieur est serré dans un cadre qui dilate la housse tubulaire auquel il est relié et qui est pourvue d'une ouverture centrale enserrant la partie stérile de l'instrument, l'ensemble améliorant l'étanchéité de cette ouverture de sortie et donc la connexion étanche entre la partie stérile de l'instrument et sa partie non stérile qui est recouverte par la housse stérile.

On peut également citer la demande de brevet US 2010/0292707 de la sté KUKA ROBOTER GMBH publiée le 18/11/2008 et qui enseigne un « dispositif de barrière/houssage stérile pour un robot chirurgical », qui est assez complexe et qui, comprenant au moins un joint tournant reliant deux organes tournant l'un par rapport à l'autre autour de l'axe commun du joint qui les relie, comporte deux housses de protection couvrant chacune un organe, les extrémités proximales des deux organes présentant chacune au moins une gorge, les extrémités proximales des deux housses recouvrant chacune la gorge de l'extrémité de l'organe que la housse correspondante protège, chaque housse étant ainsi couplée au membre qu'elle protège et seulement à lui et que ces housses ne se recouvrent jamais

### Résumé de l'invention

Le problème posé et résolu par la présente invention est ainsi de réaliser le houssage stérile d'un joint tournant entre deux parties d'un appareil médical, protégées elles-mêmes par ce houssage, liées entre elles par ce joint tournant et tournant l'une par rapport l'autre sur plus d'un tour complet, et cela sans risque de découvrir le joint tournant.

L'invention propose pour répondre à ce problème un dispositif de houssage stérile et de joint tournant entre deux organes liés, tournant l'un par rapport à l'autre et recouverts chacun par au moins une housse de protection et tel que :
- le joint tournant reliant les extrémités proximales des deux organes présente au moins une gorge entre ces deux organes, et comme décrit ci-après et représenté sur les figures jointes ladite gorge peut être formée par le joint lui-même ou être portée par celui-ci,
- l'extrémité proximale d'une première housse est apte à recouvrir non seulement l'extrémité proximale de l'organe qu'elle protège mais également au moins une partie longitudinale de la gorge du joint tournant et suivant les modes de réalisation la totalité de la longueur de cette gorge et même une partie de l'extrémité de l'autre organe,
- l'extrémité proximale d'une deuxième housse est apte à recouvrir l'extrémité proximale de l'autre organe qu'elle protège ainsi qu'une partie de l'extrémité proximale de la première housse qu'elle enserre au niveau du joint tournant dans la gorge que celui-ci présente entre les deux organes.

Les extrémités proximales des deux housses se chevauchent donc et recouvrent toutes deux ainsi partiellement ou totalement le joint tournant tout en étant indépendantes de mouvement l'une par rapport à l'autre, ce qui donne une totale liberté à ce dernier pour effectuer des rotations sans contrainte sur plusieurs tours.

Dans un mode préférentiel de réalisation, pour prévenir toute rétraction des housses qui exposerait également les bras non stériles à l'environnement, l'ouverture de l'extrémité proximale de la deuxième housse comporte un lacet réglable, qui peut être aussi un élastique, et qui est apte à assurer la permanence de contact entre les deux housses sur une partie de leur zone de recouvrement et au niveau du joint tournant: l'extrémité proximale de la deuxième housse assure ainsi une étanchéité relative mais suffisante pour protéger l'environnement contre la non stérilité des organes et est apte à tourner autant de fois que voulu autour de l'extrémité proximale de la première housse en glissant sur celle-ci au niveau donc du joint tournant, les deux organes pouvant alors effectuer plus d'un tour complet l'un par rapport à l'autre tout en restant protégé par le houssage.

Selon un mode de réalisation de l'invention, le joint tournant a une dimension transversale extérieure plus petite que celle de l'organe ayant lui-même une plus petite dimension transversale extérieure par rapport à l'autre organe, soit dans le cas de bras de robot chirurgicaux ayant des formes longitudinales cylindriques de révolution, cette dimension est le diamètre du joint tournant qui est donc inférieur à celui du plus petit diamètre des deux bras, ce qui créé une gorge entre les deux organes.

Selon un autre mode possible de réalisation de l'invention, le joint tournant a ses dimensions transversales extérieures, comme son diamètre dans le cas de forme cylindrique de révolution, égales ou supérieures à celles des organes ou bras et comporte alors une gorge perpendiculaire à l'axe de rotation de ceux-ci.

Quel que soit ainsi le mode de réalisation de ladite gorge, les extrémités proximales des deux housses, recouvrant au moins le joint tournant, se chevauchent donc dans cette gorge et s'y trouvent d'autant plus maintenues que l'extrémité proximale de la deuxième housse comporte un lacet réglable, qui peut être aussi un élastique, qui enserre cette extrémité proximale dans le fond de la gorge, ce qui a pour conséquence d'éviter tout glissement longitudinal des housses qui sinon risquerait que le joint tournant ne soit plus positionné dans la zone de recouvrement des deux housses.

Ainsi le problème posé précédemment est bien résolu par la présente invention dont les avantages évoqués ci-dessus et les solutions apportées en prouvent l'intérêt, suivant des caractéristiques spécifiques, telles que décrites plus en détail ci-après suivant le mode de réalisation présenté. La description et les figures ci-jointes en donnent en effet des exemples de réalisation mais d'autres modes de réalisation sont possibles dans le cadre de la portée de la présente invention.

### Brève description des figures

D'autres caractéristiques et avantages de l'invention apparaitront au cours de la lecture de la description détaillée qui va suivre pour la compréhension de laquelle on se reportera aux dessins annexés dans lesquels :
[Fig. 1] - la figure 1 est une vue schématique en coupe longitudinale d'un premier exemple de houssage d'un premier exemple de joint tournant suivant l'invention et reliant deux bras cylindriques de révolution comme ceux d'un robot chirurgical.
[Fig.2] - la figure 2 est une vue schématique en coupe longitudinale d'un deuxième exemple de houssage d'un deuxième exemple de joint tournant suivant l'invention et reliant deux bras cylindriques de révolution comme ceux d'un robot chirurgical ;
[Fig.3] - la figure 3 est une vue schématique en coupe longitudinale d'un troisième exemple de houssage du même deuxième exemple de joint tournant suivant l'invention que sur la figure 2.

### Description de l'invention

Dans la description de l'invention et pour la compréhension des revendications, on notera que l'invention porte sur l'ensemble et la combinaison du houssage stérile et du joint tournant entre deux organes liés, tournant l'un par rapport à l'autre et recouverts par le houssage, et que la qualification de proximale désigne les parties des éléments situées du côté du joint tournant et la qualification de distales les parties éloignées de celui-ci.

L'axe XX' de rotation des organes tournant l'un par rapport à l'autre est représenté comme étant leur axe longitudinal commun mais les axes de ces organes pourraient faire un angle entre eux et le joint tournant qui les relie pourrait former un coude.

On notera également que le joint tournant peut être soit un organe distinct des organes ou bras qu'il relie entre eux soit une composante d'un organe ou bras ou même être composé de deux éléments faisant parti chacun d'un organe ou bras Dans la description qui va suivre, des éléments identiques, similaires ou analogues seront désignés par les mêmes chiffres de référence.

### Exemples de réalisations

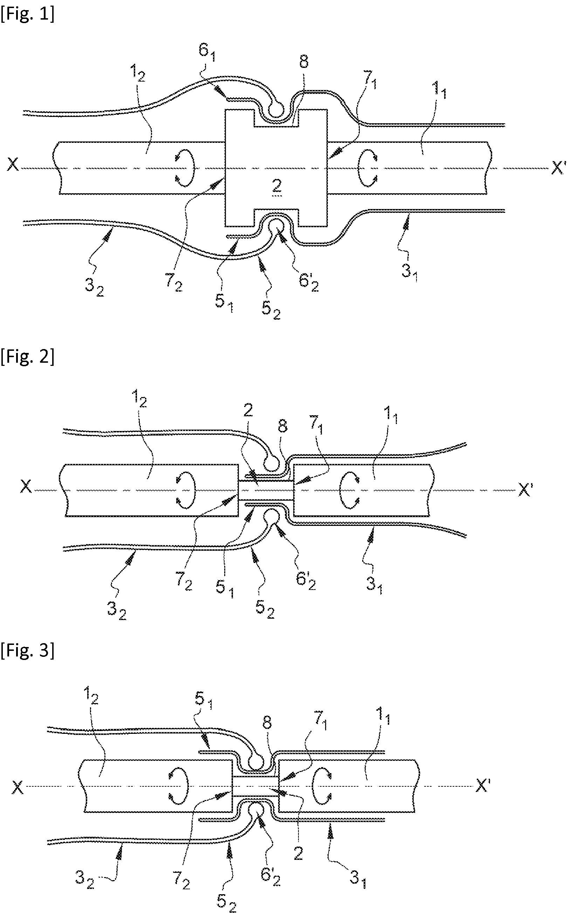

Quel que soit le mode de réalisation, dont 3 exemples sont donc représentés sur les 3 figures jointes, le dispositif de houssage stérile et de joint tournant 2 entre deux organes 1 liés, tournant l'un par rapport à l'autre et recouverts chacun par au moins une housse 3 de protection est tel que, suivant l'invention :
- le joint tournant 2 reliant les extrémités proximales 7 des deux organes (considérés donc, dans les exemples de réalisation décrits ci-après et ci-joints, comme des bras cylindriques d'un robot chirurgical), présente au moins une gorge 8 entre les deux organes 1,
- l'extrémité proximale 5₁ d'une première housse 3₁ est apte à recouvrir non seulement l'extrémité proximale 7, de l'organe (ou bras) 1₁ qu'elle protège mais également au moins une partie longitudinale de la gorge 8 du joint tournant 2,et même, comme représenté sur les trois figures cette extrémité proximale 5₁de cette première housse 3₁ recouvre la totalité de la longueur (suivant l'axe XX') de la gorge 8 du joint tournant 2 et de plus, comme représenté sur la figure 3,cette extrémité proximale 5₁ de cette première housse 3₁peut être apte à recouvrir également une partie de l'extrémité proximale 7₂ de l'autre organe 1₂,
- l'extrémité proximale 5₂ d'une deuxième housse 3₂ est apte à recouvrir l'extrémité proximale 7₂ de l'autre organe (ou bras) 1₂ qu'elle protège ainsi qu'une partie de l'extrémité proximale 5₁ de la première housse 3₁ qu'elle enserre au niveau du joint tournant 2 dans la gorge que celui-ci présente entre les deux organes (ou bras) 1.

Les deux organes (bras) 1 étant aptes à effectuer plus d'un tour complet l'un par rapport à l'autre, l'extrémité proximale 5₂ de la deuxième housse 3₂ est apte ainsi à tourner autant de fois que voulu autour de l'extrémité proximale de la première housse 3₁ en glissant sur celle-ci au niveau du joint tournant 2.

Dans des modes de réalisation particuliers, le joint tournant 2 a au moins une dimension transversale extérieure plus petite que celle de l'organe 1 ayant lui-même une plus petite dimension transversale extérieure par rapport à l'autre organe, de telle façon que le joint tournant 2 lui-même forme au niveau au moins de cette dimension transversale la dire gorge 8 ;ainsi, dans le cas de bras 1 de robot chirurgical, ayant des formes longitudinales cylindriques de révolution comme suivant les exemples des figures 2 et 3 où de plus les bras sont représentés comme étant de même diamètre, cette dimension est le diamètre du joint tournant 8 qui est donc inférieur à celui du plus petit diamètre des deux bras1 soit ici en l'occurrence celui du diamètre des deux bras, ce qui créé une gorge 8 entre les deux organes (bras) 1.

Suivant d'autres modes de réalisation, tel que représenté sur la figure 1, le joint tournant 2 comporte lui-même une gorge 8 perpendiculaire à l'axe de rotation XX' de ceux-ci et du coup un tel joint tournant 2 peut avoir des dimensions transversales (comme ici son diamètre) supérieures à celles des deux organes (bras) 1.

Dans ces exemples de modes de réalisation où les deux organes 2 sont des bras cylindriques d'axe XX' tournant l'un par rapport à l'autre suivant cet axe, les housses 3 sont tubulaires et sont aptes à être enfilées chacune sur un des bras 2 par l'extrémité distale de celui-ci (non représentée sur les figures).

Comme indiqué précédemment, afin de prévenir toute rétraction des housses 3, l'ouverture 6₂' de l'extrémité proximale 5₂ de la deuxième housse 3₂ comporte de préférence un lacet réglable apte à assurer la permanence de contact entre les deux housses 3 sur une partie de leur zone de recouvrement, soit dans la gorge 8 au niveau du joint tournant 2, et ce lacet réglable peut être un élastique.

Ainsi la première housse 3₁, qui est donc de préférence tubulaire, comme c'est généralement le cas, et qui a deux ouvertures 6 opposées à ses deux extrémités:
- s'emmanche (soit de la droite vers la gauche des figures dans les exemples de réalisation représentées dans celles-ci) par une de ses ouvertures (et qui sera celle dite proximale 6₁, une fois mise en place) sur un des organes 1₁, que l'on considère dans les exemples de réalisation décrits comme étant un premier bras cylindrique d'un robot chirurgical, à partir de l'extrémité distale (non représentée et située au-delà de la droite des figures) de celui-ci,
- recouvre ce premier bras 1₁ qu'elle protège, puis tout ou partie du joint de liaison 2 rotatoire avec l'autre organe 1₂, que l'on considère comme étant le deuxième bras cylindrique du robot chirurgical lié au premier bras 1₁ par le joint de liaison 2, et
- et vient recouvrir éventuellement (comme représenté sur la figure 3) une portion de l'extrémité proximale 7₂ de ce deuxième bras 1₂,
puis une deuxième housse 3₂, tubulaire et à deux ouvertures 6' opposées également, s'emmanche elle aussi (de la droite vers la gauche), par une de ses ouvertures (et qui sera celle distale une fois mise en place, mais non représentée et située au-delà de la gauche des figures), à partir de la même extrémité distale du premier bras 1₁ que la première housse 3₁ et par-dessus celle-ci (car l'extrémité distale, non représentée, du deuxième bras 1₂ est fixée généralement sur un support qui ne permet pas d'emmancher une housse par cette extrémité) puis est enfilée, en passant autour du joint tournant 2, sur le deuxième bras 1₂ pour le protéger et jusqu'à ce que l'extrémité proximale 5₂ de cette deuxième housse 3₂ se positionne en avant de l'extrémité proximale 7₂ du deuxième bras 1₂, en recouvrant alors partiellement ou totalement le joint tournant 2 que l'ouverture 6₂' de cette deuxième housse 3₂ enserre par tout moyen comme un lacet réglable et qui peut être élastique.

## Revendications

1. Dispositif de houssage stérile et de joint tournant (2) entre deux organes (1) liés, tournant l'un par rapport à l'autre et recouverts chacun par au moins une housse (3) de protection apte à recouvrir l'extrémité proximale (7) de l'organe (1) qu'elle protège, et comportant au moins une gorge (8), **caractérisé en ce que** :
- c'est le joint tournant (2) reliant les extrémités proximales (7) des deux organes (1) qui présente la au moins gorge (8) entre les deux organes (1),
- l'extrémité proximale (5₁) d'une première housse (3₁), qui est apte à recouvrir l'extrémité proximale (7₁) de l'organe (1₁) qu'elle protège et apte également à recouvrir au moins une partie longitudinale de la gorge (8) du joint tournant (2)
- l'extrémité proximale (5₂) d'une deuxième housse (3₂) qui est apte à recouvrir l'extrémité proximale (7₂) de l'autre organe (1₂) qu'elle protège, est apte à recouvrir également une partie de l'extrémité proximale (5₁) de la première housse (3₁) qu'elle enserre au niveau du joint tournant (2) dans la gorge que celui-ci présente entre les deux organes (1).

2. Dispositif suivant la revendication 1 **caractérisé en ce que** les deux organes (1) étant aptes à effectuer plus d'un tour complet l'un par rapport à l'autre, l'extrémité proximale (5₂) de la deuxième housse (3₂) est apte à tourner autant de fois que voulu autour de l'extrémité proximale (5₁) de la première housse (3₁) en glissant sur celle-ci au niveau du joint tournant (2).

3. Dispositif suivant l'une quelconque des revendications 1 et 2 **caractérisé en ce que** l'extrémité proximale (5₁) de la première housse (3₁) est apte à recouvrir au moins la totalité de la longueur de la gorge (8) du joint tournant (2).

4. Dispositif suivant la revendication 3 **caractérisé en ce que** l'extrémité proximale (5₁) de la première housse (3₁) est apte à recouvrir une partie de l'extrémité proximale (7₂) de l'autre organe (1₂),

5. Dispositif suivant l'une quelconque des revendications 1 à 4 **caractérisé en ce que** le joint tournant (2) reliant les extrémités proximales (7) des deux organes (1) a au moins une dimension transversale extérieure plus petite que celle de l'organe ayant lui-même une plus petite dimension transversale extérieure par rapport à l'autre organe, de telle façon que le joint tournant (2) forme lui-même au niveau au moins de cette dimension transversale ladite gorge (8).

6. Dispositif suivant l'une quelconque des revendications 1 à 4 **caractérisé en ce que** le joint tournant (2) comporte une gorge (8) perpendiculaire à l'axe de rotation de ceux-ci

7. Dispositif suivant l'une quelconque des revendications 1 à 6 et concernant deux organes (2) qui sont des bras cylindriques d'axe XX' tournant l'un par rapport à l'autre suivant cet axe **caractérisé en ce que** les housses (3) sont tubulaires et sont aptes à être enfilées chacune sur un des bras (2) par l'extrémité distale de celui-ci.

8. Dispositif suivant l'une quelconque des revendications 1 à 7 **caractérisé en ce que** l'ouverture (6₂') de l'extrémité proximale (5₂) de la deuxième housse (3₂) comporte un lacet réglable apte à assurer la permanence de contact entre les deux housses (3) sur une partie de leur zone de recouvrement dans la gorge (8).

9. Dispositif suivant la revendication 8 **caractérisé en ce que** le lacet réglable est un élastique.

## Patentansprüche

1. Vorrichtung zum sterilen Umhüllen und mit einem Drehgelenk (2) zwischen zwei verknüpften Mitteln (1), die sich relativ zueinander drehen und jeweils durch mindestens eine Schutzhülle (3) bedeckt sind, die geeignet ist, das proximale Ende (7) des Mittels (1), das sie schützt, zu bedecken, und mindestens eine Nut (8) aufweist, **dadurch gekennzeichnet, dass:**
- es das Drehgelenk (2) ist, das die proximalen Enden (7) der zwei Mittel (1) verbindet, das die mindestens eine Nut (8) zwischen den zwei Mitteln (1) aufweist,
- das proximale Ende (5₁) einer ersten Hülle (3₁), die geeignet ist, das proximale Ende (7₁) des Mittels (1₁), das sie schützt, zu bedecken, und die auch geeignet ist, mindestens einen Längsteil der Nut (8) des Drehgelenks (2) zu bedecken,
- das proximale Ende (5₂) einer zweiten Hülle (3₂), die geeignet ist, das proximale Ende (7₂) des anderen Mittels (1₂), das sie schützt, zu bedecken, geeignet ist, auch einen Teil des proximalen Endes (5₁) der ersten Hülle (3₁) zu bedecken, die sie an dem Drehgelenk (2) in der Nut, die dieses zwischen den zwei Mitteln (1) aufweist, umschließt.

2. Vorrichtung nach Anspruch 1, **dadurch gekennzeichnet, dass** die zwei Mittel (1) geeignet sind, mehr als eine vollständige Umdrehung relativ zueinander auszuführen, das proximale Ende (5₂) der zweiten Hülle (3₂) geeignet ist, so oft wie gewünscht um das proximale Ende (5₁) der ersten Hülle (3₁) gedreht zu werden, indem es auf diesem an der Drehverbindung (2) gleitet.

3. Vorrichtung nach einem der Ansprüche 1 und 2,
**dadurch gekennzeichnet, dass** das proximale Ende (5₁) der ersten Hülle (3₁) geeignet ist, mindestens die gesamte Länge der Nut (8) des Drehgelenks (2) zu bedecken.

4. Vorrichtung nach Anspruch 3, **dadurch gekennzeichnet, dass** das proximale Ende (5₁) der ersten Hülle (3₁) geeignet ist, einen Teil des proximalen Endes (7₂) des anderen Mittels (1₂) zu bedecken.

5. Vorrichtung nach einem der Ansprüche 1 bis 4,
**dadurch gekennzeichnet, dass** das Drehgelenk (2), das die proximalen Enden (7) der zwei Mittel (1) verbindet, mindestens eine äußere Querabmessung besitzt, die kleiner als die des Mittels ist, das selbst eine kleinere äußere Querabmessung in Bezug auf das andere Mittel besitzt, so dass das Drehgelenk (2) selbst an mindestens dieser Querabmessung die Nut (8) ausbildet.

6. Vorrichtung nach einem der Ansprüche 1 bis 4,
**dadurch gekennzeichnet, dass** das Drehgelenk (2) eine Nut (8) senkrecht zu der Rotationsachse davon aufweist.

7. Vorrichtung nach einem der Ansprüche 1 bis 6 und bezüglich zweier Mittel (2), die zylindrische Arme mit einer Achse XX' sind, die sich relativ zueinander entlang dieser Achse drehen, **dadurch gekennzeichnet, dass** die Hüllen (3) rohrförmig sind und geeignet sind, jeweils auf einen der Arme (2) durch das distale Ende davon aufgesteckt zu werden.

8. Vorrichtung nach einem der Ansprüche 1 bis 7,
**dadurch gekennzeichnet, dass** die Öffnung (6₂') des proximalen Endes (5₂) der zweiten Hülle (3₂) ein verstellbares Band umfasst, das geeignet ist, den permanenten Kontakt zwischen den zwei Hüllen (3) auf ihrem Teil ihres Bedeckungsbereichs in der Nut (8) zu gewährleisten.

9. Vorrichtung nach Anspruch 8, **dadurch gekennzeichnet, dass** das verstellbare Band ein Gummiband ist.

## Claims

1. Sterile covering device with a rotating joint (2) between two attached members (1), rotating relative to each other and each covered by at least one protective cover (3) capable of covering the proximal end (7) of the member (1) protected by this cover (1), and comprising at least one groove (8), **characterized in that:**
- it is the rotating joint (2) connecting the proximal ends (7) of the two members (1) which has the at least one groove (8) between the two members (1),
- the proximal end (5₁) of a first cover (3₁), which is capable of covering the proximal end (7₁) of the member (1₁) that it protects, is also capable of covering at least one longitudinal portion of the groove (8) of the rotating joint (2),
- the proximal end (5₂) of a second cover (3₂), which is capable of covering the proximal end (7₂) of the other member (1₂) that it protects, is also capable of covering a portion of the proximal end (5₁) of the first cover (3₁) that it encloses at the rotating joint (2) in the groove that the latter has between the two members (1).

2. Device according to claim 1, **characterized in that,** with the two members (1) being capable of performing more than one complete revolution relative to each other, the proximal end (5₂) of the second cover (3₂) is capable of rotating as many times as desired around the proximal end (5₁) of the first cover (3₁), sliding thereon at the rotating joint (2).

3. Device according to any one of claims 1 and 2, **characterized in that** the proximal end (5₁) of the first cover (3₁) is capable of covering at least the entire length of the groove (8) of the rotating joint (2).

4. Device according to claim 3, **characterized in that** the proximal end (5₁) of the first cover (3₁) is capable of covering a portion of the proximal end (7₂) of the other member (1₂).

5. Device according to any one of claims 1 to 4, **characterized in that** the rotating joint (2) connecting the proximal ends (7) of the two members (1) has at least one outer transverse dimension which is smaller than the outer transverse dimension of the member having itself a smaller outer transverse dimension relative to the other member, in such a way that the rotating joint (2) itself forms said groove (8), at least in this transverse dimension.

6. Device according to any one of claims 1 to 4, **characterized in that** the rotating joint (2) includes a groove (8) which is perpendicular to the axis of rotation thereof.

7. Device according to any one of claims 1 to 6 and relating to two members (2) which are cylindrical arms of axis XX' rotating relative to each other along this axis, **characterized in that** the covers (3) are tubular and each cover is capable of being threaded on one of the arms (2) by the distal end thereof.

8. Device according to any one of claims 1 to 7, **characterized in that** the opening (6₂') from the proximal end (5₂) of the second cover (3₂) includes an adjustable lace capable of ensuring permanent contact between the two covers (3) over a portion of their overlapping area in the groove (8).

9. Device according to claim 8, **characterized in that** the adjustable lace is an elastic.
